Europäisches Patentamt

(19) European Patent Office

Office européen des brevets

(11) Publication number: **0 325 847**

**A1**

(12) **EUROPEAN PATENT APPLICATION**

(21) Application number: 88311327.6

(22) Date of filing: 30.11.88

(51) Int. Cl.⁴: **A61K 39/395 , G01N 33/68 , G01N 33/574 , G01N 33/564 , C12P 21/00**

The title of the invention has been amended (Guidelines for Examination in the EPO, A-III, 7.3).

(30) Priority: 30.11.87 US 126991

(43) Date of publication of application:
02.08.89 Bulletin 89/31

(84) Designated Contracting States:
AT BE CH DE ES FR GB GR IT LI LU NL SE

(71) Applicant: **IDEC, INC.**
**11099 North Torrey Pines Road**
**La Jolla California 92037(US)**

(72) Inventor: **Kohler, Heinz**
**3132 Caminito Richardo Encinitas**
**California 92024(US)**
Inventor: **Raychaudhuri, Symal**
**3716 Carmel View**
**San Diego California 92130(US)**

(74) Representative: **Goldin, Douglas Michael et al**
**J.A. KEMP & CO. 14, South Square Gray's Inn**
**London WC1R 5EU(GB)**

(54) **Method for the selection of anti-idiotype antibodies, and their use in the diagnosis, monitoring and treatment of disease.**

(57) A selective anti-idiotype therapies of cancer, infectious disease or autoimmune disease, allergy or transplantation rejection. Prognostic idiotopes useful in disease diagnosis and/or monitoring are (i) identified and (ii) induced or suppressed by the therapeutic application of modulatory anti-idiotype antibodies, alone or in combination with other immunomodulating agents. Modulatory anti-idiotype antibodies and methods for making and using them in immunotherapy are claimed.

Modulatory anti-idiotype antibodies may be made and used in active or passive immunotherapy according to the following method. First, idiotype bearing antibodies (Ab1s) are generated or acquired. Second, select at least one idiotype bearing Ab1 or T-cell which is reactive with a cancer, infectious agent or autoimmune disease target antigen, allergen or transplant antigen for future screening. Next, make at least one anti-id (Ab2) against the Ab1 or T-cell receptor. At this point, the anti-ids (Ab2s) made are screened. Each of the anti-ids (Ab2s) may be screened either against the B or T-cell idiotopes of either the sera, blood or tissue of a statistically significant panel of patients or against the serum, blood or tissue of a single patient or animal. In either case, the purpose of the screening is to identify a correlation between idiotope level and either (i) disease presence or progression, or (ii) disease absence or regression. Finally, that anti-id (Ab2) or combination of anti-ids (Ab2s) which induces either (1) suppression of idiotopes which normally fall with disease regression; (2) stimulation of idiotopes which normally rise with disease regression; (3) stimulation of idiotopes which normally fall with disease progression; or (4) suppression of idiotopes which normally rise with disease progression are administered.

## Method And Means For Selection of Anti-Idiotype Antibodies And Their Use For The Diagnosis, Monitoring, Treatment And/or Prevention Of cancer, Autoimmune Or Infectious Disease

### Field Of The Invention

This invention is in the field of active and passive immunotherapies. More particularly, this invention is in the art of selecting idiotypes or anti-idiotypes which will be useful in the diagnosis, monitoring, therapy and/or prevention of cancer, autoimmune or infectious disease, allergy or transplant rejection.

### Background Of The Invention

### Introduction

Antigens are substances which can induce an immune response. One typical immune response is antibody production. Antibodies are produced by B cell lymphocytes. Another typical response is the stimulation of T-cells. (T-cells are another type of lymphocyte which have undergone differentiation in the thymus gland.) T-cells are capable of destroying cells infected by certain types of viruses or other pathogens. T-cells also are involved in antibody production by B-cells. Accordingly, an antigen can react either with antibodies or with receptors on the T-cells which it stimulates. Antibodies and T-cell receptors are specific for the inducing antigen.

Antibodies and T-cell receptor molecules possess variable regions which are responsible for specific antigenic recognition. The region of the antigen that actually binds to the antibody or T-cell receptor is termed the antigenic determinant or "epitope." The variable regions of antibodies and T-cell receptors contain determinants, or "idiotopes" which also are immunogenic and initiate anti-idiotype ("anti-id") immune responses.

More particularly, idiotopes are believed to be associated with the variable regions of antibody and T-cell receptors. These variable regions confer antibody and T-cell receptor specificity for antigens. Idiotopes are immune system markers on antibodies, B cell immunoglobulin receptors and T-cell receptors. An idiotype is immunologically defined by reactivity with more than one anti-idiotopic antibody which recognizes an idiotopic determinant or idiotope within a given idiotype; i.e., an idiotype is made up of a collection of idiotopes. Idiotopes are best defined by their binding to monoclonal anti-idiotypic antibodies.

Idiotopes can be found on more than one different group of antibodies or T-cell receptors; i.e., idiotopes may be shared by different antibodies or T-cell receptors from different individuals. This characteristic is referred to as a "public" idiotope. Antibodies also may have "private" idiotopes expressed uniquely by a single individual. An individual will display both public and private idiotopes. Hence, anti-id therapies which detect public idiotopes may be applied for the treatment of multiple patients whereas anti-ids directed against private idiotopes would be applied in patient specific therapy. It also should be noted that idiotopes are distinct from isotypic (immunoglobulin class-specific), xenotypic (species specific) and allotypic (certain species sup-group specific) determinants.

Each antibody and T-cell receptor has at least one paratope which is the binding site for an antigen determinant termed an "epitope." A paratope may serve as an idiotope, i.e., the paratope may stimulate an anti-idiotypic response in which, like the original antigen, the anti-idiotypic antibodies bind to the paratope. If an anti-paratope anti-idiotope antibody structurally mimics the antigen it is called the "internal image" of the antigen. In addition to these antiparatopic anti-ids which represent the configuration of the original antigen, other anti-id antibodies define antibody and T-cell receptor idiotopes which participate in the regulation of immune responses. These idiotypes are termed regulatory idiotypes and they are not necessarily "internal images" of the original antigen. For a general discussion of these background principles, see Burdette, S. and Schwartz, R., New Eng. J. of Med. 317:219 (1987).

### The Idiotypic Network

In any given species or individual, the number of different antibodies and T-cell receptors is very large ($>10^7$). Each antibody and T-cell receptor expresses several idiotopes which are linked through a regulatory network to complementary anti-idiotopes. Jerne, N., Ann. Immunol. 1256:373 (1974), proposed that the immune system is regulated by a network composed of idiotypes and anti-idiotypes. There is a large amount of experimental evidence indicating that the function of the idiotypic network is to maintain the homeostasis of the immune system and to regulate immune responses. As idiotypes are expressed by B-cells and T-cells, the idiotypic network encompasses both humoral (antibody) and cellular (T-cell mediated) immune responses. Specific immune responses are regulated by specific anti-idiotypic antibodies either on a one to one basis, or in groups through shared idiotopes. These anti-idiotypic antibodies are termed "regulatory anti-idiotypes" and they do not necessarily represent the internal image of the target antigen. See, Goldberg, B., et al., J. Exp. Med. 158:515 (1983).

Anti-Idiotype Therapy

Recently, the use of "internal image" anti-idiotypes in therapy has been suggested and demonstrated in several experimental systems (EPO App. No. 84810516.9, filed Oct. 25, 1984, Pub. No. 0141783; Nisonoff, A., and Lamoyi, E., Clin. Immunol. Immunopathol. 21:397 (1981); Kennedy, R.C., et al., Biotechniques 3:4040 (1985)). These internal image anti-idiotypes have been used as surrogates for antigens in generating specific immune responses against viral, bacterial and parasitic infections and cancers (Herlyn, D. et al., Science 232:100 (1986); Raychaudhuri, S. et al., J. Immunol. 137:1743 (1986)).

Currently, internal image polyclonal and monoclonal anti-idiotypes are being used for active immunotherapy in animals and man. The process of generating such internal image anti-idiotypes is as follows: 1) an antibody is made against a tumor antigen or an infectious agent. This antibody is called "Ab1". 2) anti-idiotypic antibodies ("Ab2s") are made against the first antibodies. These anti-ids (Ab2s) are screened for the expression of idiotopes which represent the configuration of the initial antigen (such as that expressed by a tumor or infectious agent). This screening consists of inhibiting the Ab1-Ab2 binding by the original antigen and the biological testing of the anti-idiotypes (Ab2) as a surrogate antigen in vivo. This testing is done in animals and has been demonstrated previously for internal image anti-idiotypes representing tumor antigens and infectious agents (Kennedy, R.C. et al., Biotechniques 3:4040 (1985); Herlyn D., et al., Science 232:100 (1986); Raychaudhuri, et al., J. Immunol. 137:1743 (1986)).

One of the potential advantages of internal image anti-ids over native antigens is that internal image anti-idiotypic hybridoma antibodies often are more economically and easily prepared than the original antigens. In addition, with respect to infectious diseases, anti-ids are free of infectious agents, and are therefore safer for the patient than other vaccines which may contain infectious particles. Another proposed advantage of the internal image approach (for example, EPO App. No. 84810516.9, filed Oct. 25, 1984, pub. No. 0141783), lies in the presentation of the relevant antigen (tumor associated or infectious agent associated) in a different molecular and biological context. This may be more relevant for cancer in which a natural state of immune tolerance to tumor antigens often exists.

Limitations of the Prior Art

However, there are severe shortcomings in the current approach using internal image anti-idiotypes. We have demonstrated that immunizations with different internal image anti-ids representing the same antigen epitope may or may not induce protective immunity. In the prior art, the status and response of the patient's non-internal image regulatory idiotopes are neither properly considered nor controlled for. These responses may connect to the regulatory idiotypic network of the patient which mediates effective anti-tumor or anti-infectious agent immune responses and controls deleterious immune responses, e.g., autoimmune or allergic responses or transplant rejection. The outcome of these regulatory idiotypic/anti-idiotypic interactions may be either beneficial or detrimental in the disease process. Thus, the methodology currently utilized in the prior art does not properly account for these regulatory idiotypic interactions in selecting anti-idiotypes for immunotherapy.

We believe, therefore, that the selection of anti-idiotypes based solely on internal image characteristics is inadequate for therapeutic purposes and that selection of anti-ids based on the correlation of idiotope levels with disease progression or regression is an important consideration in the design of effective idiotype immunotherapy. It is an object of the present invention to remove elements of serendipity from the development of therapeutically useful anti-idiotypic antibodies.

For example, using what is presently known in the field, the administration of an internal image or non-internal image antibody (which has been selected only by virtue of its reactivity with an Ab1 antibody) may

3

stimulate in the patient a regulatory circuit which activates suppressor T-cells, thereby weakening anti-tumor immunity. In another disease, autoimmunity, the activation of suppressor T-cells may be beneficial for the patient, inhibiting the destruction of normal tissues by the immune system. Thus, it is evident from the above considerations that regulatory idiotopes play an important role in determining the outcome of anti-idiotypic immunotherapy. The prior art does not teach now to identify and to control for the benefit of patients, the biological effects of idiotopes which correlate with disease progression or regression and which are induced by the disease process and by therapeutic interventions. Another object of this invention is to deal with the deficiencies of current techniques and to propose an approach which will more consistently induce the desired beneficial immune responses in anti-idiotype immunotherapy.

Summary Of The Invention

The present invention is a selective anti-idiotype therapy or prevention of cancer, infectious disease and autoimmune disease, allergy or transplant rejection. The selection of anti-idiotypic agents is based on screening such agents over time against patient idiotopes associated with disease progression or regression, these idiotopes herein termed "prognostic idiotopes". Similarly, T helper cells recognizing idiotopes or anti-idiotopes as a part of the regulatory network undergo changes in frequency and activation during disease progression or regression. Detection of idiotopes on antibodies, or B- or T-cell receptors which are associated with the immune response to a given disease is a useful adjunct, as taught herein, to a rational and effective approach to immunotherapy. This selection will insure that idiotopes associated with the host immune response to disease are manipulated in specific ways to support the host immunity against the disease.

The present invention lies in the recognition of a correlation between the expression of certain idiotopes in the sera, blood or tissues of patients with cancer, infection, autoimmune diseases, allergy or transplant rejection and the host's ability to combat these disorders. The expression of such idiotopes are monitored with sets of monoclonal anti-idiotypic antibodies. The present invention also lies in the use of such anti-idiotypes in diagnosis, monitoring and immunotherapy. We have found that idiotopes useful in disease modulation are produced by therapeutic application of effective anti-idiotype antibodies, these antibodies herein termed "modulatory anti-idiotype antibodies". We disclose and claim such modulatory anti-idiotype antibodies as well as a method for making them and using them in disease diagnosis, monitoring, prevention and therapy.

Modulatory anti-idiotype antibodies may be used in active or passive immunotherapy according to the following method. First, idiotype bearing antibodies (Ab1s) are generated or acquired. Second, select at least one idiotype bearing Ab1 or T-cell which is reactive with a cancer, infectious agent or autoimmune disease target antigen, allergen or transplant antigen for future screening. Next, make at least one anti-id (Ab2) against the Ab1 or T-cell receptor. At this point, the anti-ids (Ab2s) made are screened. Each of the anti-ids (Ab2s) may be screened either against the B- or T-cell idiotopes present in the sera, blood or tissues of a statistically significant panel of patients or against idiotopes expressed in the serum, blood or tissues of a single patient or animal. In either case, the purpose of the screening is to identify a correlation between idiotope level and either (i) disease presence or progression, or (ii) disease absence or regression. Finally, that anti-id (Ab2) or combination of anti-ids (Ab2s) which induces either (1) suppression of idiotopes which normally fall with disease regression; (2) stimulation of idiotopes which normally rise with disease regression; (3) stimulation of idiotopes which normally fall with disease progression; or (4) suppression of idiotopes which normally rise with disease progression are administered. These methods and the modulatory anti-idiotypes of this invention can be utilized in conjunction with other anti-id and non-anti-id immunotherapies or other therapies.

This invention also may be practiced in disease diagnosis, monitoring and/or prevention. For example, using this invention, a panel of anti-idiotypes to clinically relevant or prognostic idiotopes may be generated. Depending on the patient idiotype profile generated, inter alia, according to the methods of this invention, Ab2s may be administered to prevent or inhibit cancer or autoimmune or infectious disease or allergy or transplant rejection.

Brief Description Of The Drawing

Fig. 1 is a graph of prognostic idiotope D11 expression over time.

4

Fig. 2 is a graph showing how the pattern of prognostic idiotope reactivities may be correlated to tumor manifestation in a hypothetical patient (Patient Smith of Example 1). Figure 2 plots idiotopes and tumor burden as manifested by serum levels of a tumor marker, carcinoembryonic antigen (CEA) over time.

Fig. 3 is a graph showing 2F10 prognostic idiotope levels in untreated tumor bearing mice and its positive correlation with animal survival. The solid vertical bar indicates time of the death of the mouse.

Fig. 4 is a graph showing the correlation of 2F10 prognostic idiotope levels with survival in tumor bearing mice treated with cyclophosphamide (hereinafter sometimes referred to as "cy").

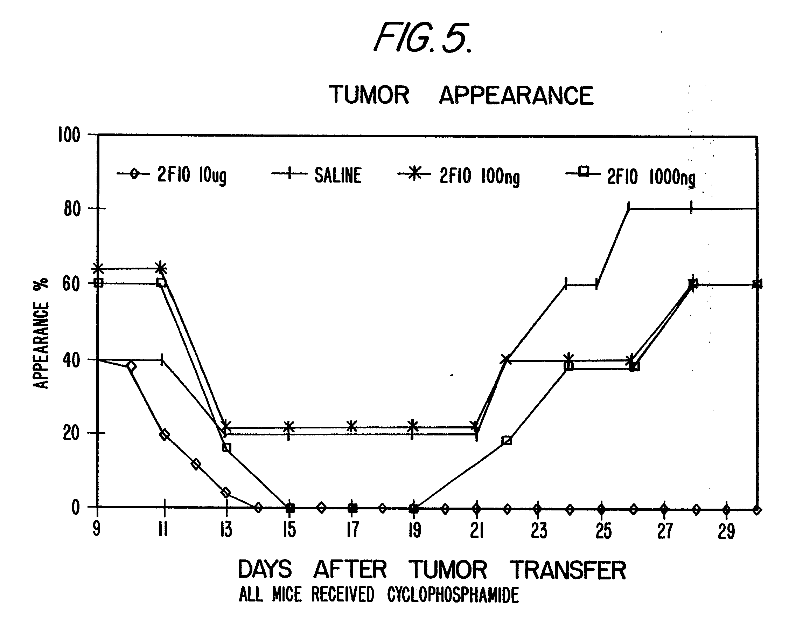

Fig. 5 is a graph showing the beneficial effects of increasing the dosage of anti-id antibody administered to decrease tumor growth in animals treated with combined anti-id and cyclophosphamide therapy. Groups of DBA/2 mice were injected intradermally with 10,000 live L1210/GZL tumor cells; on day 3 they received cyclophosphamide (80 mg/kg); on day 7 they were injected intravenously with saline or with different doses of an anti-idiotype antibody, anti-2F10, which is reactive with prognostic idiotope 2F10 in such a way as to stimulate said prognostic idiotopes. Tumor appearance was monitored by palpation.

Detailed Description of Preferred Embodiments

A. Definitions

The following terms, as used in this disclosure and claims, are defined as follows:

passive immunotherapy: the administration of antibody; passive transfer of antibody to a patient or animal.

active immunotherapy: the induction of antibody and/or T-cell response in a patient or animal.

suppression: the inhibition of an immune response or the inhibition of the expression of an idiotypic response.

stimulation: the induction of an immune response or the induction of the expression of an idiotypic response.

tumor regression: an overall 30% or greater reduction of tumor.

prognostic idiotope: an antigen binding or regulatory idiotope of an antibody or B- or T-cell receptor which increases or decreases over time with disease progression or which increases or decreases over time with disease remission.

modulatory anti-idiotype antibody: an anti-idiotype antibody which can be used in vivo to stimulate or suppress levels of prognostic idiotopes.

All of the references cited in this disclosure are hereby incorporated by reference.

B. Introduction

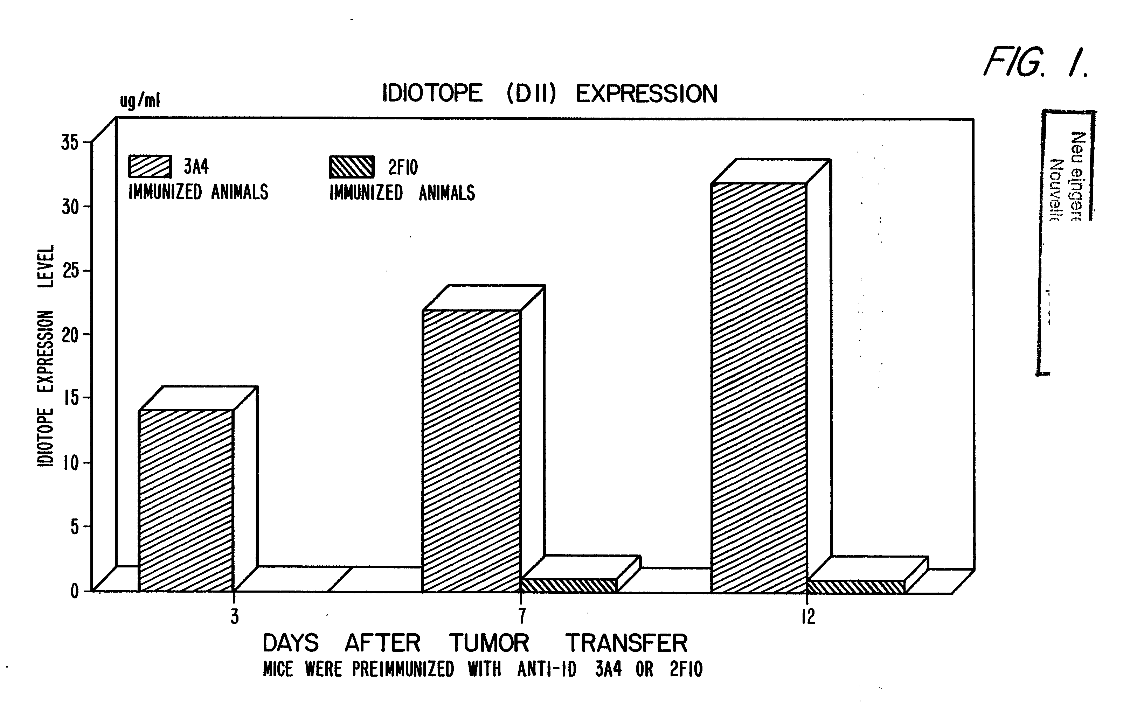

Recently it was observed in an animal tumor system that the expression of idiotopes in the sera of tumor bearing mice correlated with the resistance of the host against the tumor. Certain idiotopes had high serum levels in animals with progressively growing tumors, and low or absent levels in animals which rejected the tumor. A similar correlation was observed in animals vaccinated with anti-idiotypic antibodies which induced or failed to induce tumor resistance. In Fig. 1 two groups of mice were immunized with monoclonal anti-idiotypic antibodies (anti-3A4 or anti-2F10) reactive with anti-tumor Ab1. Two weeks later both groups received live tumor cells. Animals immunized with one, anti-3A4 displayed accelerated tumor growth and high serum levels of prognostic idiotope D11. Animals immunized with anti-2F10 displayed retarded tumor growth and low serum levels of prognostic idiotope D11. The level of the D11 prognostic idiotope was measured using a monoclonal anti-idiotypic antibody, anti-D11, recognizing an idiotope on an anti-tumor Ab1. The amount of prognostic idiotope D11 is expressed in micrograms/ml serum.

We presently disclose that (i) suppression of idiotopes which can be shown to increase over time with disease progression and/or which can be shown to decrease over time with disease regression; and/or (ii) stimulation of idiotopes which can be shown to increase over time with disease regression and/or which can be shown to decrease over time with disease progression is beneficial.

C. Methods

The first step is to identify idiotypic components associated with the patient's (or animal's) response to

5

EP 0 325 847 A1

tumor or disease. This will be accomplished by any of several approaches known to those skilled in the art. See e.g., Rapp, et al., Cancer Res., 43:2592 (1983) and Wittner, M.K. et al., J. Immunol., 128:595 (1982) (hereby incorporated by reference). For example, antibodies (Ab1s, or T-cells may be obtained from the patient or experimental animals with specificity for the relevant tumor or disease antigen, from commercial sources, or by methods routinely practiced by those skilled in the art. For purposes of this invention the Ab1 may be either polyclonal or monoclonal antibodies. Polyclonal antibodies can be isolated from patients' or animals' sera and affinity purified using immobilized disease associated antigens as described by Real, F.X., et al., J. Exp. Med. 160:1219 (1984) and in the Handbook of Experimental Immunology(ed. Weir, D.M., et al., 1986)(hereinafter referred to as "the Handbook"). Monoclonal antibodies can be generated by standard somatic cell hybridization rescue fusion ("rescue fusion") using standard techniques practiced in the prior art (see the Handbook, supra and Carrol, W.L., et al., J. Immunol. Meth. 80:61 (1986)) with or without in vitro stimulation techniques, and utilizing patients' peripheral B cells, or any other B-cells, e.g. those derived from lymph nodes, spleen or bone marrow which may be sensitized to relevant disease associated antigen.

Polyclonal and monoclonal antibodies can also be generated using the techniques described above from human subjects or experimental animals inoculated with tumor, with tumor extracts or disease associated antigens or infectious disease vaccines. T-cells specific for relevant disease associated antigens can be cloned from accessible lymphocytes which may be obtained from peripheral blood, lymph nodes, spleen or other tissues as described in detailed by Slovin, S.F., et al., J. Immunol. 137:3042 (1986). These Ab1 antibodies or T-cells reacting with tumor or disease associated antigens can be used as immunogens to generate a panel of monoclonal anti-idiotypes. For suitable methods for antibodies see Raychadhuri, S. et al., J. Immunol. 137:1743 (1986) and for T-cells see Ert1, H.C., et al., J. Exp. Med. 159:1776 (1985). The anti-ids (Ab2s) will be characterized with respect to the expression of internal image and non-internal image idiotopes using standard assays routinely practiced in the art as described in detail by Raychaudhuri, S., et al., J. Immunol. 137:1743 (1986).

The second step is to screen patients' sera or peripheral blood or tissue for the presence and absence of B- and T-cell idiotopes reacting with the panel of anti-idiotypes. That is, the reactivity of each monoclonal anti-id (Ab2) will define serum and lymphocyte idiotopes and their respective levels. The idiotope serum screen will be performed using radioimmunoassay, enzyme-linked immunoassay or equivalent assay as described in detail by Raychaudhuri, S. et al., J. Immunol. 137:1743 (1986). Briefly, to determine the concentration of idiotopes (Ab1 ids) in the test serum, polyvinylchloride plates are coated with 200-400ng of different monoclonal anti-idiotype antibodies overnight at 4 degrees Celsius. The plates are washed twice with phosphate buffer saline and then blocked with 1% bovine serum albumin for one hour. Thereafter, the plates are incubated with different dilutions of test sera or known Ab1 antibody standards and 20,000 cpm of 125-I-labelled Ab1 overnight at 4 degrees Celsius. The plates are washed and counted in a gamma counter.

Peripheral blood or tissue lymphocyte idiotope expression will be assayed by routine immunofluorescence techniques as described in Kohler, H., et al., Science 186:643 (1974) or by routine immunohistochemical techniques as described in the Handbook cited supra. Briefly, in the immunofluorescence method, the mononuclear cell fraction is purified by Ficoll- (R Phairacia Fine Chemicals) Hypaque (R Winthrop Laboratories) density gradient centrifugation. Monocytes are removed by adherence to plastic. The cells are then incubated with the different monoclonal anti-ids (Ab2s) and control antibodies. The cells are washed and then incubated with an appropriate fluorochrome labelled secondary antibody. The percentage of T-cells is then assessed by either a cytofluorograph or fluorescense microscope.

The patient will be monitored using the anti-idiotype panel during the course of the disease beginning at the time of diagnosis. For each anti-idiotype antibody, the level of reactivity with serum and/or lymphocyte idiotopes will be plotted against time and correlated (see below) with disease progression or regression.

The third step is to establish a correlation of serum or lymphocyte idiotope expression and the disease state. The correlations of idiotope expression with disease state will be determined by analyzing serum and lymphocytes as described above from specimens obtained (i) at diagnosis; (ii) before, during and following prophylaxis or successful therapy (including surgery, chemotherapy, radiation therapy, immunotherapy, vaccination, etc.) resulting in complete or partial disease prophylaxis or regression; (iii) during periods of disease progression; (iv) from normal individuals without disease. Serum and lymphocyte idiotopes which correlate with the presence or progression of disease or which correlate with the absence, regression or prevention of disease (i.e., prognostic idiotopes) will be identified.

The fourth step is to select anti-idiotypes for either active or passive immunotherapy. In the present invention, anti-ids (Ab2s) which bind to serum or lymphocyte prognostic idiotopes in a patient which (1) increase with disease absence or regression or prevention; or (2) decrease with disease progression will be

6

used for active stimulatory immunization. Also, in the present invention anti-ids (Ab2s) which bind to serum or lymphocyte idiotopes in patients which increase with disease presence or progression; or (2) decrease with disease regression will be used in suppressive immunotherapy. The administration of some anti-ids will be shown to exert a beneficial therapeutic effect. others may not have a therapeutic effect and will not be used in vivo, however, measurement of their reactivity with serum idiotopes may be useful in disease diagnosis and monitoring.

Alternatively, antibodies bearing those idiotopes which increase with disease progression can be utilized for active immunization to induce active suppression (i.e., to induce anti-idiotypes which suppress). Antibodies bearing these idiotopes will be isolated from serum by anti-idiotypic affinity chromatography employing standard techniques practiced in the art using immobilized anti-idiotypic antibodies (see the Handbook, supra) or by rescue fusion employing B-lymphocytes from patients with progressive disease and any of several suitable fusion partner cell lines described or available in the prior art.

The principle of the above approaches is to select among different antibodies bearing idiotopes those which will be used as active immunizing agents to enhance beneficial immune responses and those which will be used to suppress harmful immune responses. The necessary information for this selection will be obtained through an idiotypic panel analysis on the patient's serum and lymphocytes using the assays described above for monitoring serum, blood and tissue idiotope levels. It should be noted that in addition to their application in selecting appropriate anti-idiotype therapy, certain serum and lymphocyte idiotope level assays may predict the clinical course and be utilized in diagnostic and monitoring assays to assess patient prognosis and to guide decisions regarding the initiation, intensification or cessation of anti-idiotype or other therapeutic interventions.

## D. Modifications

It may be necessary to use T-cell idiotopes (Slovin, S.F., et al., J. Immunol. 137:3042 (1986); Ertl, H.C., et al., J. Exp. Med. 159:1776 (1985); Raychaudhuri, S. et al., J. Immunol. 139:2096 (1987)) or Ab1s obtained by rescue fusion (Carrol, W.L., et al., J. Immunol. Meth. 89:61 (1986)) or isolated using affinity purification (Real, F.X., J. Exp. Med. 160:1219 (1984)) from multiple time points in the disease course, for example when patients are in remission or during disease progression. Alternatively, one could use an already existing monoclonal or polyclonal antibody (Ab1) which has been used in passive immunotherapy for producing the panel of Ab2 anti-idiotypic monoclonal antibodies. In addition, Ab2 anti-idiotypic antibodies may be obtained by rescue fusions or by affinity purification (see references cited in this paragraph) from patients or animals treated with existing monoclonal or polyclonal antibodies (Ab1) or other therapies.

An alternative method which may be useful for identifying idiotopes associated with disease states is to purify them from the sera of diseased patients using the immunoglobulin fraction employing standard affinity chromatography methods with immobilized anti-immunoglobulins. (See the Handbook cited supra). These immunoglobulins would be utilized to generate anti-ids (Ab2s) as described by Raychaudhuri, S., et al., J. Immunol. 137:1743 (1986) in mice made tolerant to human immunoglobulin isotopic determinants as previously described in the prior art. (See the Handbook cited supra). The methods also may be practiced without the tolerization step. The resulting anti-ids (Ab2s) would be screened against a panel of serum samples from individuals with different disease states (progressive disease, regressing disease, normal individuals) as described in Section c. Methods, step 3.

It is understood that for purposes of this invention all anti-ids (Ab2s) will have been demonstrated by testing against a panel of representative antibodies not to cross react with xenotypic, isotypic or allotypic determinants. Assays for this purpose are readily performed by those skilled in the art and useful assays for this purpose are described in the Handbook cited supra.

## E. Treatment of Mice with Anti-Id Antibody Anti-2F10

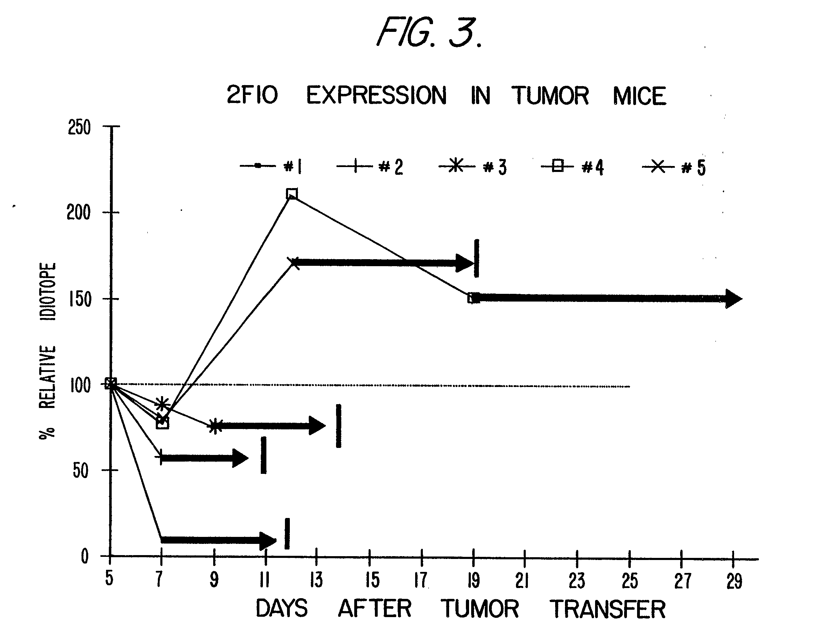

To illustrate the use of anti-ids in therapy, we have monitored the serum levels of the prognostic idiotope 2F10 in mice. First, we measured the steady state level of prognostic idiotope 2F10 in five mice. For each mouse, an internal standard of 100% was established. On day 0, we injected the mice with 1000 live L1210/GZL tumor cells. Thereafter, we monitored the level of idiotope 2F10 in each mouse. In mice numbered 1, 2 and 3, illustrated in Figure 3, the level of idiotope 2F10 was initially suppressed. After a few days, the 2F10 idiotope level was less than 100% of the initial level. In mice numbered 4 and 5, the idiotope level initially decreased but then substantially increased. The three mice (mice 1, 2 and 3) with sustained low levels of prognostic idiotope 2F10 died within 11-14 days. Mice 4 and 5 which showed increases in prognostic idiotope 2F10 above the 100% internal standard lived 19 days and beyond 29 days, respectively. We therefore conclude that increasing levels of prognostic idiotope 2F10 in individual mice correlate with the ability of these mice to resist tumor and that therapeutic interventions which increase 2F10 levels would increase the survival of tumor bearing animals.

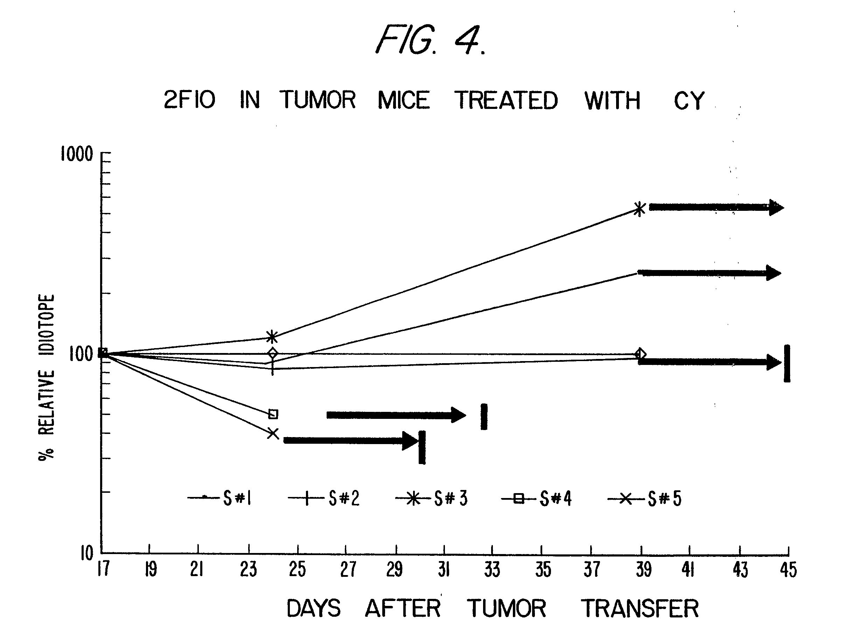

We also monitored prognostic idiotope 2F10 expression in mice treated with cyclophosphamide. Mice were injected with 1000 live L1210/GZL tumor cells on day 0 and 2F10 idiotope levels were monitored thereafter. Mice were injected with 80 mg/kg body weight cyclophosphamide 17 days after transplantation of the tumor cells and the level of 2F10 idiotope in each mouse arbitrarily was set at 100% on day 17 (See Fig. 4). Idiotope 2F10 levels were monitored throughout. In the cases shown in Figure 4, in mice 1 and 3 prognostic idiotope 2F10 levels increased following cyclophosphamide treatment. In mice 2, 4 and 5 shown in Figure 4, prognostic idiotope levels remained the same or decreased following cyclophosphamide treatment. Mice 1 and 3, having increased levels of prognostic idiotope 2F10 following cyclophosphamide treatment were alive 45 days following tumor transfer. Mice 2, 4 and 5, having prognostic idiotope 2F10 levels that either remained the same or decreased following cyclophosphamide treatment died within 45 days after tumor transfer. This experiment shows the prognostic value of monitoring certain idiotopes in patients or animals receiving therapy. As a control for the prognostic value of idiotope 2F10 we monitored the expression of an unrelated idiotope. Our results show that monitoring an unrelated idiotope ("F6-3", an idiotope related to the phosphorylcholine immune response) in the same individual mice, shows no correlation with survival.

We also have examined the therapeutic effect of a modulatory anti-idiotype, anti-2F10 (an anti-idiotype antibody which binds to prognostic idiotope 2F10) in tumor bearing mice. Groups of five mice were injected intravenously with different doses of anti-2F10 from 0.1 to 1000 micrograms/kg body weight. The highest percentage of surviving mice was observed with a dose of anti-2F10 in the amount of 50 micrograms/kg body weight (80% survival on day 17, 60% on day 20, 40% on day 28 and 0% on day 45). At lower or at higher doses of anti-2F10 the survival rate was lower at each of these time points. For example, at treatment doses of 0.1 micrograms/kg body weight and at 1000 micrograms/kg body weight no mice survived beyond 17 days.

To demonstrate that combined anti-id and cyclophosphamide treatment was superior to either alone, we performed an additional experiment. In this experiment, we monitored prognostic idiotope 2F10 levels and survival in mice treated with cyclophosphamide and anti-2F10 antibody. Tumor cells were injected as in the previous examples. On day 17, after idiotope 2F10 levels had stabilized in each mouse, we administered cyclophosphamide (80 mg/kg). Two days later mice were injected with anti-2F10 antibody in the amount of 10 micrograms intravenously. In contrast to the experiments described above in which animals were treated either with cyclophosphamide or anti-2F10 alone, all mice that received both cyclophosphamide and anti-id therapy survived beyond 45 days. 2F10 idiotope levels increased in all of these mice. In one case a 400% increase was observed. These data demonstrate that treatment of tumor with a combination of anti-idiotype and cyclophosphamide was superior to treatment with either agent alone.

In other studies, combined therapy with cyclophosphamide (80 mg/kg) and anti-2F10 decreased the incidence of tumor growth following adoptive tumor transfer. Groups of DBA/2 mice were injected intradermal with 10,000 live L1210/GZL tumor cells; on day 3 they received cyclophosphamide (80 mg/kg); on day 7 they were injected i.v. with saline or with different doses of anti-2F10 or saline. Tumor appearance was monitored by palpation.

All groups treated with anti-2F10 had a lower incidence of tumor growth 30 days after tumor transfer compared to the control group injected with saline. Tumors were not detected 15 days after tumor transfer in any of the animals treated with the higher dose (10 micrograms per mouse) of anti-2F10 (Figure 5). Thus, increasing the dose of anti-id administration was beneficial in reducing tumor growth. It will be appreciated by those skilled in the art that stimulation of idiotopes may be achieved either (1) through active immunization, for example, intramuscular, subcutaneous, intradermal or intraparitoneal administration of immunogens with or without carriers and/or adjuvants; or (2) through intravenous administration of antibodies at low doses without adjuvants or carriers; and that suppression of idiotopes may be achieved through passive immunotherapy using high doses of antibodies without adjuvants or carriers administered intravenously or intraparitoneally.

The foregoing experiments indicate that the best modes of therapy are those which appropriately modulate idiotope levels known to correlate with the clinical course. As the specific experiments illustrated, mice treated with combined cyclophosphamide and anti-id therapy showed enhanced prognostic 2F10 idiotope levels which correlated with anti-tumor effects and survival. In other instances, anti-id treatment alone or anti-id therapy combined with other treatment modalities may have equivalent or superior effects on modulating relevant prognostic iodiotype levels and subsequent clinical results. In all of these cases, the sera, blood or tissue prognostic idiotope levels will be measured over time in a series of patients to identify those therapies which appropriately modulate prognostic idiotope levels correlating with disease progression or regression.

The preceding data illustrates that the anti-idiotype antibody anti-2F10 which we have identified using the methods of this invention is useful in treatment of tumors. Tumor bearing mice treated with anti-2F10 and cyclophosphamide showed increased survival. The use of cyclophosphamide alone was successful in extending survival beyond 45 days in only two out of five animals; however, when anti-2F10 antibody was administered in conjunction with cyclophosphamide all five animals tested lived beyond 45 days of tumor transfer.

The methods herein described involving serum levels of prognostic idiotopes also may be practiced with T-cell bearing prognostic idiotopes. In this approach, the frequency of peripheral blood T-cells, hematopoietic tissue T-cells, or other tissue T-cells bearing specific idiotopes is determined by standard methods (Raychadhuri, S. et al., J. Immunol. 139:2096 (1987); Slovin, S.F. et al., J. Immunol. 137:3042 (1986); Ert1, H.C. et al., J. Exp. Med. 159:1776 (1985)). The levels of these idiotope positive T-cells may be determined as referenced above using 1) tumor antigens or antibodies reactive with tumor antigens; or 2) tissue antigens involved in autoimmunity or antibodies reactive with these autoantigens; or 3) antigens expressed by infectious pathogens or antibodies reactive with these infectious agents; or 4) allergens or antibodies reactive with these allergens; or 5) transplantation antigens or antibodies reactive with these transplantation antigens; or 6) anti-idiotype antibodies derived from any of the antigen reactive antibodies in 1) through 5) above. Subsequently, the frequency of these idiotope specific T-cells is examined over time to identify the T-cell idiotopes which correlate with disease progression or regression. Therapeutic interventions are then chosen or designed on the basis of their ability to do any one of the following: 1) suppress idiotopes which normally fall with disease regression; 2) stimulate idiotopes which normally rise with disease regression; 3) stimulate idiotopes which normally fall with disease progression; or 4) suppress idiotopes which normally rise with disease progression.

We have demonstrated that the levels of certain idiotope recognizing helper T-cells correlate with the progression or regression of tumor growth. For example, the frequency of T helper cells expressing idiotopes defined by reactivity with the anti-L1210/GZL tumor antibody 2B2 was increased in animals preimmunized subcutaneously with the nonprotective anti-idiotype antibody 3A4 compared to animals preimmunized subcutaneously with the protective anti-idiotype antibody 2F10 (as described above) who had a lower frequency of 2B2 idiotype positive helper T-cells. The results are set forth in the following Table 1.

TABLE 1

| TUMOR BEARING ANIMALS PREIMMUNIZED WITH: | TUMOR STATUS | FREQUENCY OF 2B2 IDIOTOPE POSITIVE T HELPER CELLS PER $10^6$ LYMPH NODE T-CELLS |
|---|---|---|
| Anti-Id 3A4 | Progression | 500 |
| Anti-Id 2F10 | Regression | 66 |

F. Treatment of Hypothetical Patients

We describe below the treatment of four hypothetical patients. The first patient (Patient Smith of Example 1) represents the approach to be taken during the initial development of anti-idiotypes to prognostic idiotopes. The second patient (Patient Jones of Example 2) represents an example of treatment after a panel of anti-idiotypes to clinically relevant prognostic idiotopes have been generated. The dosages, routes of administration and formulation and monitoring of therapy set forth in Example 2 are equally applicable to the treatment of Patient Smith of Example 1. Example 1 sets forth in greater detail how the prognostic idiotopes comprising the broad panel are identified and produced in the first instance. Significant panel data would require analysis of at least a few hundred patients with the target disease and controls comprised of healthy individuals and patients with other diseases.

1. Patient Smith

Mr. Smith has been diagnosed with colorectal cancer. The treatment of choice is surgical removal of the tumor. Before surgery serum samples should be obtained and cryopreserved, also peripheral white blood cells should be cryopreserved and lymph node biopsy or surgically removed lymph nodes should be secured and cryopreserved. All cryopreserved cells are frozen with liquid nitrogen in dimethyl sulfoxide ("DMSO") according to standard practices for maintaining cellular viability. These specimens will be used to obtain antibodies reactive with colorectal carcinoma associated antigens (Ab1 antibodies).

In the next steps Ab1 antibodies will be utilized to generate anti-Id Ab2s. Several approaches may be taken to obtain Ab1s reactive with colorectal cancers. Immunoglob ulins from the patient's serum reactive with colorectal carcinomas may be obtained by affinity chromatography employing immobilized tumor extracts utilizing standard techniques routinely practiced by those skilled in the art, for example, as described by Real, F.X. et al., J. Exp. Med. 160:1219 (1984), and the Handbook cited supra. In addition, the patient's tumor draining lymph nodes may be employed in somatic cell hybridization rescue fusions and the resulting antibodies screened for their reactivity with tumor cell lines or tissues as described in the Handbook cited supra. The methods for the generation and specificity screening of these somatic cell hybridoma Ab1 antibodies are routinely performed by those skilled in the art. See the Handbook cited supra. In addition, Ab1 polyclonal and monoclonal antibodies may be generated in animals with specificity for colorectal tumor associated antigens utilizing methods routinely practiced by those skilled in the art.

Ab1 antibodies produced by any one of these methods may be used to generate in Balb/c mice anti-idiotypic monoclonal antibodies employing standard methods as described in Raychaudhuri, S., et al., J. Immunol. 137: 1743 (1986). These anti-id antibodies were selected on the basis of their reactivity with the corresponding Ab1 and the absence of reactivity with xenotype and allotype and isotype control antibodies in RIA or ELISA assays as described previously in Section C. Methods, and by Raychaudhuri S., et al., J. Immunol. 137:1743 (1986).

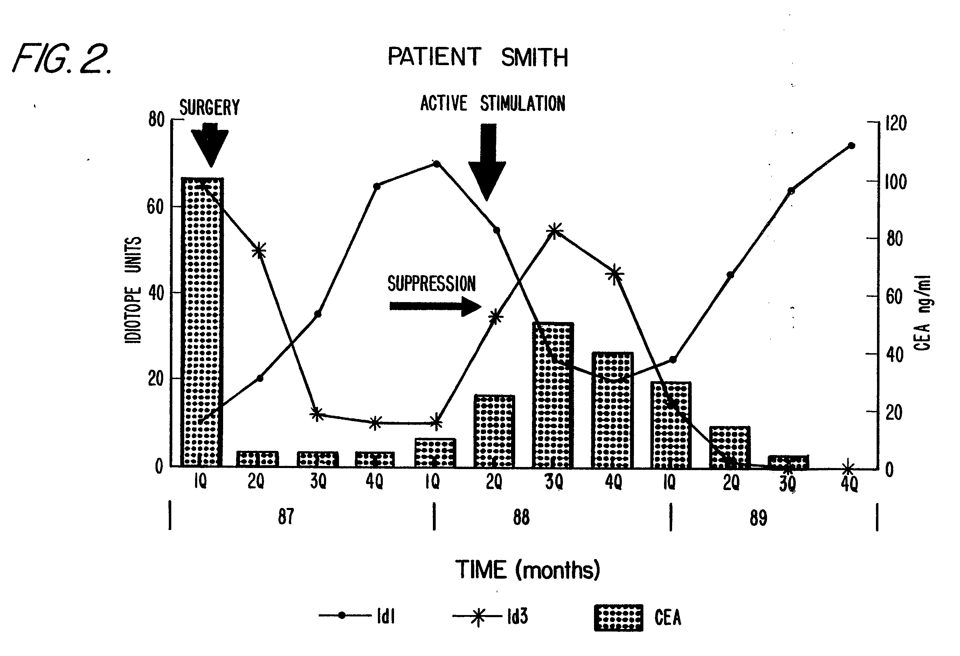

Serum samples collected from the patient Smith over the 12 month time period from diagnosis were assayed for binding to the different anti-idiotype monoclonal antibodies (Ab2s) using the idiotope monitoring assay described in Section c. Methods, and by Raychaudhuri, S., et al., J. Immunol. 137:1743 (1986), and routinely practiced by those skilled in the art. Numerous anti-Ids (Ab2s) were used to screen the serum samples. Two anti-idiotypes showed different levels of binding to complementary serum idiotopes over time. These prognostic idiotope levels correlated (idiotope 1 correlated inversely, and idiotope 3 correlated directly) with tumor burden as measured by the serum level of tumor marker e.g., carcinoembryonic antigen (CEA) and/or by physical examination or radiographic examination detecting tumor metastasis in the liver. By the end of the first post surgery year, prognostic idiotope 1 had reached a high level in the serum, while prognostic idiotope 3 was very low. By the middle of post surgery year 2, radiographic computerized axial tomography (CAT) scans and CEA levels indicated relapse of the tumor. Idiotype panel analysis at this time indicated a drop in prognostic idiotope 1 and rise of prognostic idiotope 3 (See Figure 2).

Because there was a clear correlation of disease progression with an increase of prognostic idiotope 3, and of disease remission with an increase of prognostic itiotope 1, modulatory anti-Id 3 was chosen for passive anti-idiotype suppression of prognostic idiotope 3, while modulatory anti-Id 1 was chosen for active anti-idiotype stimulation of prognostic idiotope 1.

The patient Smith received four intravenous infusions of 500 mg each of modulatory anti-Id 3 (Ab2) at weekly intervals over a month period following passive immunotherapy protocol described for Patient Jones (See Example 2, infra). one week later active immunization was initiated using modulatory anti-Id 1 (Ab2) in an adjuvant formulation (also see the protocol for Patient Jones) intramuscular (im) at a dose of 500 micrograms to 5 mg. Ten days later the patient received a booster immunization using the same dose and formulation.

Prognostic idiotope 3 level started to decrease during the passive immunotherapy protocol, while prognostic idiotope 1 began to rise following the active immunotherapy protocol. After the completion of therapy, the CEA level started to decrease. The CAT scan showed improvement.

The colorectal patient is given as an example for illustrating the principle in the disclosed immunotherapy using prognostic idiotopes and modulatory anti-idiotype antibodies. It is not intended to restrict the use of this approach to colorectal cancer or to cancer alone. Furthermore, this example represents an early developmental stage in the disclosed method. It is expected that a panel of modulatory anti-idiotypes will be made which will be representative and encompassing the prognostic idiotopes in a large cohort of patients suffering from the same cancer or autoimmune or infectious disease or allergy or transplant rejection. To exemplify the approach taken during more advanced states of immunotherapies examples of other hypothetical patients with cancer, autoimmune and infectious disease are given below.

2. Patient Jones

Mr. Jones is found to have a skin lesion which reveals upon biopsy a malignant melanoma. The treatment of choice is wide surgical excision of the tumor. Prior to surgery, a peripheral blood specimen is analyzed for serum and B- and T-lymphocyte idiotope levels which are known to correlate with disease activity and response to therapy. These assays are performed as described in Sections C, D, and E of this application, utilizing anti-idiotypic monoclonal antibodies generated against anti-melanoma antibodies derived by affinity purification from melanoma patients. Mr. Jones is found to have high serum levels of prognostic idiotope 4 which has been shown to correlate in other patients with progressive disease activity and a poor prognosis. In addition, Mr. Jones was found to have low serum levels of prognostic idiotope 5 and low T-lymphocyte levels of prognostic idiotope 6. Previous studies have indicated that high levels of prognostic idiotope 5 and prognostic idiotope 6 correlate with disease regression and a good prognosis.

Following surgical removal of the tumor, the patient is treated by passive immunotherapy with modulatory anti-Id 4 to suppress the idiotope response associated with disease progression. In addition, the patient is immunized (active immunotherapy) with modulatory anti-Id 5 and modulatory anti-Id 6 to increase the idiotope responses associated with disease regression. For suppressive passive immunotherapy, Mr. Jones received at least four weekly intravenous infusions of 500mg of murine modulatory anti-Id 4 in a physiologic buffered saline solution and received additional weekly treatments until the serum levels of prognostic idiotope 4 decreased to levels found in normal subjects. One week later active immunization was initiated using murine modulatory anti-Id 5 and murine modulatory anti-Id 6 (0.5mg of each) in an adjuvant formulation.

Active immunizations were administered subcutaneously or intramuscularly every other week for at least three cycles or until an increase in levels of prognostic idiotope 5 and prognostic idiotope 6 were demonstrated utilizing the previously described idiotope assays (see, sections C, D and E). Anti-Id adjuvant formulations utilizing either Syntex Adjuvant Formulation -1 (SAF-1) (see, Allison, A.C., Bio/technology 5:1041 (1987) or alum adjuvants were employed as described below following the coupling of the anti-Id proteins to keyhole limpet hemocyanin (KLH) according to the method of Racychadhuri, S. et al., J. Immunol. 137:1743 (1986).

To phosphate buffer saline containing 0.4% v/v Tween 80 (R ICI America) add 5% v/v Pluronic L121 (R BASF) and 10% v/v squalane. Vortex this mixture vigorously to produce a uniform emulsion. N-acetylmuramyl-L-threonyl-D-isoglutamine should then be added and the adjuvant-vehicle mixture mixed thoroughly. One volume of this mixture should be added to an equal volume of KLH-coupled anti-Id solution (2mg/ml) in phosphate buffer saline and vortexed briefly to ensure complete mixing. The final concentrations of the components should be 0.17% Tween 80, 2.5% Pluronic L121 and 5% squalane.

For the alum adjuvants, alum precipitation should be performed as follows. Equal volumes of anti-Id KLH (1.0 mg/ml) and alum (9% w/v) should be mixed, adjusted to pH 6.5 and allowed to precipitate for 30 minutes. The precipitate should be collected by centrifugation (750 x g, 5 min.) and resuspended in phosphate buffer saline to yield 0.5 mg/ml anti-Id-KLH.

The patient should be immunized with 0.5 mg to 5 mg anti-idiotype protein coupled to an equivalent amount by weight of KLH in either of the indicated adjuvant formulations (total volume 0.5 to 2.0 ml) and boosted two weeks later, using the same amount of antigen. Patients immunized with modulatory anti-Id in SAF-1 should receive a dose of 1.0 mg N-acetylmuramyl-L-threonyl-D isoglutamine in the primary vaccination and 1.0 mg in subsequent booster immunizations.

Following this treatment protocol, prognostic idiotope 4 levels were within normal limits and prognostic idiotope 5 and prognostic idiotope 6 levels had increased significantly over pre-treatment values. The patient remained asymptomatic for one year. During this interval idiotope level testing demonstrated an increase in prognostic idiotope 4 levels and a decrease in prognostic idiotope 5 and prognostic idiotope 6 levels. Standard blood chemistries revealed abnormalities in liver enzymes. CAT scan of the abdomen revealed the presence of a lesion in the liver consistent with metastatic melanoma. Liver biopsy confirmed metastatic melanoma. The initial course of passive immunotherapy with modulatory anti-Id 4 and active immunotherapy with modulatory anti-Id 5 and modulatory anti-Id 6 was repeated. Two months after the cessation of therapy, prognostic idiotope 4 levels had returned to normal values, prognostic idiotopes 5 and 6 levels were elevated and repeat CAT scan of the liver demonstrated marked reduction in tumor size.

3. Patient Doe

Ms. Doe has been diagnosed with myasthenia gravis. This is an autoimmune disease with the major manifestation being muscular weakness secondary to antibody destruction of acetylcholine receptors which participate in muscular contraction. A peripheral blood specimen is analyzed for eerum and B- and T-lymphocyte idiotope levels which are known to correlate with disease activity and response to therapy. These assays were performed as described in the Sections C, D, and E, utilizing anti-idiotypic monoclonal antibodies generated against anti-acetylcholine receptor antibodies. The anti-acetylcholine antibodies were obtained from human patients or animals employing affinity purification or somatic cell hybridization techniques with sensitized lymphocytes as routinely practiced by those skilled in the art. Ms. Doe is found to have high serum levels of prognostic idiotope 7 which has been shown in other patients to correlate directly with disease activity. In addition, Ms. Doe is found to have low serum levels of prognostic idiotope 8 and low T-lymphocyte levels of prognostic idiotope 9. Previous studies have indicated that high levels of prognostic idiotopes 8 and 9 correlate with disease remission. Employing identical passive immunotherapy and active immunotherapy anti-Id protocols as described for Patient Jones, MS. Doe is treated by passive immunotherapy with modulatory anti-Id 7 to suppress the idiotope response associated with disease activity. In addition, the patient is immunized (active immunotherapy) with modulatory anti-Id 8 and modulatory anti-Id 9 to increase the idiotope responses associated with disease remission. One month after the completion of this therapy, the patient notices increased muscular strength. Six months after treatment, the patient returns for medical follow-up and describes the onset of mild muscular weakness. Repeat testing of serum and lymphocyte idiotope levels reveals an identical pattern of increased prognostic idiotope 7 associated with disease activity and low levels of prognostic idiotopes 8 and 9 associated with disease remission. The patient is re-treated with the same passive and active immunotherapy protocol as described above, resulting in the abatement of muscular weakness.

### 4. Patient Wright

Mr. Wright has recovered from acute hepatitis induced by hepatitis B virus ("HBV") infection. A portion of patients infected with this virus go on to develop chronic active hepatitis or hepatocellular carcinoma. A peripheral blood specimen is analyzed for serum and B- and T-lymphocyte idiotope levels which are known to correlate with different stages of disease activity induced by infection with the hepatitis B virus. These assays were performed as described in Sections C, D and E, utilizing anti-idiotypic monoclonal antibodies generated against Ab1 antibodies and/or T-cell receptors associated with different stages of disease induced by HBV infection, i.e., acute hepatitis, chronic carrier state, chronic active hepatitis, hepatocellular carcinoma. Mr. Wright is found to have high serum levels of prognostic idiotope 10 which has been shown to correlate in other patients with the development of chronic active hepatitis in HBV infected individuals. In addition, Mr. Wright was found to have low serum levels of prognostic idiotope 11. Previous studies have indicated that high levels of this idiotope correlate with remission of chronic active hepatitis in HBV infected individuals.

Mr. Wright presents for medical follow-up with the classic signs and symptoms of chronic active hepatitis. The patient then is treated by passive immunotherapy with modulatory anti-Id 10 to suppress the idiotope response associated with the development or progression of chronic active hepatitis. In addition, the patient is immunized (active immunotherapy) with modulatory anti-Id 11 to increase the idiotope response associated with the remission of chronic active hepatitis. The therapeutic protocols for these passive and active immunotherapies are identical to those described for Patient 2, Mr. Jones.

Further evaluation of Mr. Wright's past medical history reveals that he was immunized with a standard vaccine available for hepatitis B virus ("HBV") a few years prior to contracting hepatitis B. Analysis of his sera revealed the absence of prognostic idiotope 15 which has been found to be present in the sera of individuals effectively immunized with the standard HBV vaccine. Analysis of other individuals who fail to mount protective immunity in response to the HBV vaccine have also been demonstrated to have low levels of prognostic idiotope 15. Active immunization of patients similar to Mr. Wright with mcdulatory anti-Id 15 employing the active immunotherapy protocols as described above result in the ability of these patients to respond to the hepatitis B vaccine with protective immunity. consequently, this modulatory anti-Id 15 is valuable for incorporation into prophylactic vaccines for hepatitis B virus.

The hypothetical treatments of these four patients are given by way of illustration, not limitation. Those skilled in medicine, immunology, molecular biology and related arts will be able to carry out this invention in the diagnosis, monitoring, treatment and/or prevention of cancer, autoimmune and infectious diseases, allergy or transplant rejection.

**Claims**

1. A method for selecting an anti-idiotype antibody or antibody fragment for the treatment or prevention of cancer, autoimmune or infectious disease, allergy or transplant rejection in a patient or animal said method comprising:

a) select at least one idiotope bearing Ab1 or T-cell, said Ab1 or said T-cell having at least one receptor means reactive with a cancer, infectious agent or autoimmune disease target antigen, allergen or transplantation antigen;

b) make at least one anti-idiotype antibody against said idiotope of either said Ab1 or said T-cell receptor means;

c) screen each of said anti-idiotype antibody(ies) against the B- or T-cell idiotopes in serum or tissue of said patient to identify at least one prognostic idiotope which correlates over time to disease progression;

d) administer at least one modulatory anti-idiotype antibody or combination thereof which induces an increase in those patient idiotopes which normally decrease with disease progression or which induces a decrease in those patient idiotopes which normally increase with disease progression.

2. A method for selecting an anti-idiotype antibody or antibody fragment for the treatment or prevention of cancer, autoimmune or infectious disease, allergy or transplant rejection in a patient or animal, said method comprising:

a) select at least one idiotope bearing Ab1 or T-cell, said Ab1 or said T-cell having at least one receptor means reactive with a cancer, infectious agent or autoimmune disease target antigen, allergen or transplantation antigen;

b) make at least one anti-idiotype antibody against said idiotope of either said Ab1 or said T-cell receptor means;

c) screen each of said anti-idiotype antibody(ies) against the B- or T-cell idiotopes in serum or tissue of said patient to identify at least one prognostic idiotope which correlates over time to disease regression;

d) administer at least one anti-idiotype antibody or combination thereof which induces a decrease in those patient idiotopes which normally decrease with disease regression or which induces an increase in those patient idiotopes which normally increase with disease regression.

3. A method of claim 1 wherein more than one modulatory anti-idiotype antibody is identified which correlates to more than one idiotope associated with disease progression.

4. A method of claim 2 wherein more than one modulatory anti-idiotype antibody is identified which correlates to more than one idiotope associated with disease regression.

5. A method for selecting an anti-idiotype antibody or antibody fragment for the treatment or prevention of cancer, autoimmune or infectious disease, allergy or transplant rejection in a patient or animal, said method comprising:

a) select at least one idiotope bearing Ab1 or T-cell, said Ab1 or said T-cell having at least one receptor means reactive with a cancer, infectious agent or autoimmune disease target antigen, allergen or transplantation antigen;

b) make at least one anti-idiotype antibody against said idiotope of either said Ab1 or said T-cell receptor means;

c) screen each of said anti-idiotype antibody(ies) against the sera, tissue or T-cells of a statistically significant panel of patients to identify at least one idiotope which correlates to disease progression;

d) administer at least one modulatory anti-idiotype antibody or combination thereof which induces an increase in those patient idiotopes which normally decrease with disease progression or which induces a decrease in those patient idiotopes which normally increase with disease progression.

6. A method for selecting an anti-idiotype antibody or antibody fragment for the treatment or prevention of cancer, autoimmune or infectious disease, allergy or transplant rejection in a patient or animal, said method comprising:

a) select at least one idiotope bearing Ab1 or T-cell, said Ab1 or said T-cell having at least one receptor means reactive with a cancer, infectious agent or autoimmune disease target antigen, allergen or transplantation antigen;

b) make at least one anti-idiotype antibody against said idiotope of either said Ab1 or said T-cell receptor means;

c) screen each of said anti-idiotype antibody(ies) against the sera, tissue or T-cells of a statistically significant panel of patients to identify at least one idiotope which correlates to disease regression;

13

d) administer at least one anti-idiotype antibody or combination thereof which induces a decrease in those patient idiotopes which normally decrease with disease regression or which induces an increase in those patient idiotopes which normally increase with disease regression.

7. The method of claim 1 or 2 or 3 or 4 or 5 or 6 wherein said anti-idiotype antibody or combination thereof is administered with agents having immunomodulatory activity.

8. The modulatory anti-idiotype antibodies made by the method of claims 1 or 2 or 3 or 4 or 5 or 6.

9. A modulatory anti-idiotype antibody of claim 8 wherein the Ab1 used to generate said anti-idiotype antibody was monoclonal.

10. A modulatory anti-idiotype antibody of claim 8 wherein the Ab1 used to generate said anti-idiotype antibody was polyclonal.

11. A modulatory anti-idiotype antibody of claim 8 wherein the Ab1 used to generate said anti-idiotype antibody was derived in whole or in part from a non-human mammalian source.

12. A modulatory anti-idiotype antibody of claim 8 wherein the Ab1 used to generate said anti-idiotype antibody was derived in whole or in part from a human source.

13. A modulatory anti-idiotype antibody of claim 8 which is monoclonal.

14. A modulatory anti-idiotype antibody of claim 8 which is polyclonal.

15. A modulatory anti-idiotype antibody of claim 8 which has been derived in whole or in part from a non-human mammalian source.

16. A modulatory anti-idiotype antibody of claim 8 which has been derived in whole or in part from a human source.

17. A method for the diagnosis and therapy or for the monitoring and therapy or prevention of illness, comprising the steps of (1) using at least one immunostimulatory agent to measure over time the level of a prognostic idiotope in a patient, the level of said prognostic idiotope which normally increases during disease regression, or which normally decreases during disease progression; and (2) using this information to guide at least one therapeutic decision regarding the commencement, continuation, cessation dose modification or withholding of the administration of said agent to said patient to increase the levels of said prognostic idiotope.

18. A method for the diagnosis and therapy or for the monitoring and therapy of illness, comprising the steps of (1) using at least one immunosuppressive agent to measure over time the increase of a prognostic idiotope in a patient, the level of said prognostic idiotope which normally increases during disease progression, or which normally decreases during disease regression; and (2) using this information to guide at least one therapeutic decision regarding the commencement, continuation, cessation, dose modification or withholding of the administration of said agent to said patient to decrease the levels of said prognostic idiotope.

19. A method for the diagnosis or monitoring of illness comprising the step of assaying over time for the amount of a prognostic idiotope known to correlate with disease progression or regression.

20. An immunostimulatory agent made by the process of claim 17.

21. An immunosuppressive agent made by the process of claim 18.

22. A method for inducing suppression of idiotopes comprising the steps of:

a) identify at least one antibody or T-cell receptor which bears at least one idiotope which increases with disease progression;

b) administer said antibody(ies) or T-cell receptor(s) in an amount and manner sufficient to stimulate immune network components so as to result in suppression of said idiotope.

# IDIOTOPE (DII) EXPRESSION

FIG. 1.

EP 0 325 847 A1

FIG.2.

PATIENT SMITH

ACTIVE STIMULATION

SURGERY

SUPPRESSION

IDIOTOPE UNITS

CEA ng/ml

1Q 2Q 3Q 4Q 1Q 2Q 3Q 4Q 1Q 2Q 3Q 4Q

87          88          89

TIME (months)

Id1        Id3        CEA

EP 0 325 847 A1

Neu eingereicht / Ne
Nouvellement dé

FIG. 3.

2F10 EXPRESSION IN TUMOR MICE

─■─ #1   ─+─ #2   ─*─ #3   ─□─ #4   ─✕─ #5

% RELATIVE IDIOTOPE

DAYS AFTER TUMOR TRANSFER

EP 0 325 847 A1

# FIG. 4.

## 2FI0 IN TUMOR MICE TREATED WITH CY

Legend: — S#1 — S#2 — S#3 — S#4 — S#5

Y-axis: % RELATIVE IDIOTOPE

X-axis: DAYS AFTER TUMOR TRANSFER

EP 0 325 847 A1

## FIG.5.

### TUMOR APPEARANCE

All mice received cyclophosphamide.

Legend: 2F10 10ug, SALINE, 2F10 100ng, 2F10 1000ng.

X axis: DAYS AFTER TUMOR TRANSFER (9 to 29).
Y axis: APPEARANCE % (0 to 100).

Neu eingereicht / Newly fil
Nouvellement déposé

EP 0 325 847 A1

European Patent Office

**PARTIAL EUROPEAN SEARCH REPORT**
which under Rule 45 of the European Patent Convention shall be considered, for the purposes of subsequent proceedings, as the European search report

Application number

EP 88 31 1327

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 113 431 (YESHIVA UNIVERSITY) <br> * Whole document * | 1-16, 19 | A 61 K 39/395 <br> G 01 N 33/68 <br> G 01 N 33/574 <br> G 01 N 33/564 <br> C 12 P 21/00 |
| X | PROC. NATL. ACAD. SCI, USA, vol. 80, February 1983, pages 850-854 <br> G. SOLOMON et al..: "Use of mono-clonal antibodies to identify shared idiotypes on human antibo-dies to native DNA from patients with systemic lupus erythematosus" <br><br> * Page 852, column 2, lines 23-40; page 853 * | 1-16, 19 | |
| X | LANCET, vol. ii, no. 7400, pages 417-422 <br> D.A. ISENBERG et al.: "Anti-DNA antibody idiotypes in systemic Lupus erythematosus" <br><br> * Whole document * | 1-16, 19 | TECHNICAL FIELDS SEARCHED (Int. Cl.4) <br><br> A 61 K <br> C 12 P <br> G 01 N |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 8-16,19
Claims searched incompletely: 1-7
Claims not searched: 17,18,20-22

Reason for the limitation of the search:

1-7, partially; 17,18,22:
Method for treatment of the human or animal body by surgery or therapy (See art. 52(4) of the European Patent Convention)
Claims 21,22 are "for" an immunostimulatory agent made by the process of claims 17 and 18 respectively. However, neither claim 17 nor claim 18 discloses a process for making an immunostimulatory agent

| Place of search | Date of completion of the search | Examiner |
|---|---|---|
| The Hague | 07-03-1989 | SKELLY |

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| X | WO-A-84 02 848 (CENTOCOR INC.)<br><br>* Whole document * | 1-16, 19 | |
| X | WO-A-87 05 703 (BOEHRINGER MANNHEIM GmbH)<br><br>* Whole document; especially page 14, line 19-31 * | 1-16, 19 | |
| X | EP-A-0 117 114 (DANA FARBER CANCER RESEARCH INST.)<br><br>* Whole document; especially page 5, line 31 - page 6, line 29 * | 1-16, 19 | TECHNICAL FIELDS SEARCHED (Int Cl.4) |
| A | BIOTECHNOLOGY, vol. 4, no. 3, March 1986, pages 210-218 New York, uS N. CHIORAZZI: "Human monoclonal antibodies as probes to study autoimmune and allergic disorders"<br><br>* Whole document, especially page 212, column 1, line 24 - column 2, line 10 * | | |
| A | SCIENTIFIC AMERICAN, vol. 255, no. 1, July 1986, pages 40-48, New York, US R.C. KENNEDY et al.: "Anti-idio-types and immunity" | | |
| A | CLINICAL IMMUNOLOGY AND IMMUNOPATHOLOGY, vol. 21, 1981, pages 397-406; Academic Press, Inc. A. NISONOFF et al. "Hypothesis: Implications of the presence of an internal image of the antigen in anti-diiotypic antibodies: Possible application to caccine production" | | |
| A | JOURNAL OF IMMUNOLOGY; vol. 126, no. 6, June 1981, pages 2397-2402 Williams & Wilkins Co. A. HATZUBAI et al.: "The use of a monoclonal anti-idiotype antibody to study the biology of a human | ./. | |

EPO Form 1505.3  06.78

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| | B cell lymphoma" | | |
| A | CHEMICAL ABSTRACTS, vol. 81,. no. 23, December 9, 1974, page 373, abstract 150069r, Columbus, Ohio, US K. EICHMANN: "Idiotype suppression. I. Influence of the dose and of the effector functions of antiidiotypic antibody on the production of an idiotyp" | | |
| A | THE JOURNAL OF IMMUNOLOGY, vol. 133, no. 4, October 1984, pages 1846-1851, The American Ass. of Immunologists, US R.S. GEHA: "Idiotypic determinants on human T cells and modulation of human T cell responses by anti-idiotypic antibodies" | | TECHNICAL FIELDS SEARCHED (Int Cl.4) |
| P,X | EP-A-0 264 911 (OTSUKA PHARMACEUTICAL CO.) <br> * Claims * | 1-16, 19 | |

--------